# EUROPEAN PATENT APPLICATION

(11) **EP 3 957 313 A1**
(43) Date of publication of application: **23.02.2022**
(21) Application number: 21191207.6
(22) Date of filing: 13.08.2021
(51) Int. Cl.: A61K 35/28, A61K 35/545, A61P 13/10, A61K 9/00, A61K 9/28

(54) **USE OF STEM CELL EXOSOMES FOR TREATING OVERACTIVE BLADDER**

(30) Priority: 14.08.2020 CN 202010817239
(71) Applicant: Shenzhen Hongji Biotechnology Co., Ltd, Shenzhen, Guangdong 518063 (CN)
(72) Inventor: JI, Guangju, Shenzhen, 518063 (CN); YANG, Zhiguang, Shenzhen, 518063 (CN); ZHU,, Keqi, Shenzhen, 518063 (CN)
(74) Representative: Bassil, Nicholas Charles

(57) **Abstract**

The disclosure relates to use of stem cell exosomes in preparing a medicament for treating overactive bladder. It has been confirmed that stem cell exosomes are effective in the treatment of overactive bladder and can be used in preparing a medicament for treating overactive bladder.

## Description

### TECHNICAL FIELD

The present disclosure relates to the technical field of stem cell, in particular to use of stem cell exosomes in preparing a medicament for treating overactive bladder.

### BACKGROUND

Overactive bladder (OAB) is a syndrome characterized by urinary urgency, often accompanied with urinary urgency, frequency and nocturia, with or without urge urinary incontinence. The International Continence Society (ICS) defines OAB in the following two aspects: 1) urodynamics: a disorder characterized by the involuntary contraction of the detrusor during the filling of bladder, accompanied by the willingness to urinate, and resulted by detrusor hyperreflexia in a neurological disease or non-neurological detrusor instability; and 2) symptomatology: a group of symptoms manifested as frequent urination, urgency urination, and urge incontinence, where no local disease factors but neurological factors that may cause symptoms are present in patients.

OAB incidence in the population is age-dependent. OAB will seriously affect the life of patients and reduce their life quality. Also, OAB will cause a great psychological burden in the patient, and trigger a series of psychological disorders, such as shame, isolation, depression, or the like, which directly affect the patient's family and work and lead to a vicious circle.

OAB is not clear in etiology, and its therapy, such as tolterodine, trosponium chloride, solifenacin, etc, is primarily focused on M receptor blockers and mainly aims at relieving symptoms. However, M receptor blockers commonly used in the treatment of OAB are less effective.

### SUMMARY

Accordingly, the present disclosure provides use of stem cell exosomes in preparing a medicament for treating overactive bladder to improve the current situation of poor effectiveness of conventional M receptor blockers used in the treatment of OAB.

It has been confirmed that stem cell exosomes are effective in the treatment of overactive bladder, can significantly improve the abnormal urination caused by urethral obstruction, and can significantly delay the abnormal bladder morphology caused by urethral obstruction.

In an embodiment, the stem cell exosomes are at least one selected from embryonic stem cell exosomes, mesenchymal stem cell exosomes, and induced pluripotent stem cell exosomes.

A medicament for treating overactive bladder comprises an active ingredient and a pharmaceutically acceptable auxiliary, the active ingredients comprising stem cell exosomes.

In an embodiment, the stem cell exosomes are humanized stem cell exosomes or murine stem cell exosomes.

In an embodiment, the medicament is in a dosage form of tablets, capsules, granules, pills, injections, or sustained-release preparations.

In an embodiment, the medicament is tablets, and the pharmaceutically acceptable auxiliary is at least one selected from the group consisting of a diluent, a binder, a wetting agent, a disintegrant, and a lubricant.

In an embodiment, the diluent is at least one selected from the group consisting of starch, dextrin, sucrose, glucose, lactose, mannitol, sorbitol, xylitol, microcrystalline cellulose, calcium sulfate, calcium hydrogen phosphate, and calcium carbonate;
and/or, the binder is at least one selected from the group consisting of starch slurry, dextrin, syrup, honey, glucose solution, microcrystalline cellulose, acacia mucilage, gelatin mucilage, sodium carboxymethyl cellulose, methyl cellulose, hydroxypropyl methyl cellulose, ethyl cellulose, acrylic resin, carbomer, polyvinylpyrrolidone, and polyethylene glycol;
and/or, the disintegrant is at least one selected from the group consisting of starch, microcrystalline cellulose, low-substituted hydroxypropyl cellulose, cross-linked polyvinylpyrrolidone, cross-linked sodium carboxymethyl cellulose, sodium carboxymethyl starch, polyoxyethylene, sorbitol, fatty acid ester, and sodium dodecyl sulfonate;
and/or, the lubricant is at least one selected from the group consisting of talc, silicon dioxide, stearate, tartaric acid, liquid paraffin, and polyethylene glycol;
and/or, the wetting agent is at least one selected from the group consisting of water, ethanol, and isopropanol.

In an embodiment, the medicament is injections, and the pharmaceutically acceptable auxiliary is at least one selected from the group consisting of a solubilizer, a pH adjusting agent, and an osmotic pressure regulator.

In an embodiment, the solubilizer is at least one selected from the group consisting of ethanol, isopropanol, propylene glycol, polyethylene glycol, poloxamer, lecithin, and hydroxypropyl-β-cyclodextrin;
and/or, the pH adjusting agent is at least one selected from the group consisting of citrate, phosphate, carbonate, acetate, hydrochloric acid, and hydroxide;
and/or, the osmotic pressure regulator is at least one selected from the group consisting of sodium chloride, mannitol, glucose, phosphate, citrate, and acetate.

### BRIEF DESCRIPTION OF THE DRAWINGS

FIG. 1 is an image showing identification of mouse embryonic stem cell exosomes prepared in Example 1 in the western blot;
FIG. 2 is an electron micrograph of mouse embryonic stem cell exosomes prepared in Example 1;
FIG. 3 is a diagram of the particle size distribution of mouse embryonic stem cell exosomes prepared in Example 1;
FIG. 4 shows the urine stains of mice in the sham operation group, the operation group, and the operation + exosome group in the urine stain test in Example 2;
FIG. 5 shows the statistical results of the number of urine stains of mice in the sham operation group, the operation group, and the operation + exosome group in the urine stain test in Example 2;
FIG. 6 shows the bladder pressure curves for one mouse in each of the sham operation group, the operation group, and the operation + exosome group in the urodynamic test in Example 2;
FIG. 7 shows statistical results of the number of bladder contractions of mice in the sham operation group, the operation group, and the operation + exosome group in the urodynamic test in Example 2;
FIG. 8 shows the degree of bladder exposure, before and after urination, of mice in the sham operation group, the operation group, and the operation + exosome group in Example 2;
FIG. 9 shows the statistical results of the wet bladder/body weight ratio of mice in the sham operation group, the operation group, and the operation + exosome group in Example 2.

### DETAILED DESCRIPTION OF THE EMBODIMENTS

In order to facilitate the understanding of the present disclosure, the present disclosure will be described below in a more comprehensive manner. The present disclosure may, however, be embodied in many different forms and should not be construed as limited to the embodiments set forth herein. Rather, these embodiments are provided so that this disclosure will be thorough and complete.

All technical and scientific terms used herein have the same meaning as commonly understood by those skilled in the technical field of the present disclosure unless otherwise defined. The terms used in the description of the present disclosure herein are for the purpose of describing particular embodiments only and are not intended to limit the present disclosure.

An embodiment of the present disclosure provides use of stem cell exosomes in preparing a medicament for treating overactive bladder.

Stem cells are a type of cells with infinite or immortal self-renewal ability, capable of producing at least one type of highly differentiated progeny cells. Stem cells are divided into embryonic stem cells, adult stem cells, and induced pluripotent stem cells (iPS cells) from their source. The embryonic stem cells are primitive, highly undifferentiated cells that can differentiate into more than two hundred types of cells under specific conditions. The adult stem cells refer to undifferentiated cells that exist in a differentiated tissue, which may selfrenew and specialize to form the cells that make up the type of tissue. The adult stem cells exist in various tissues and organs of a body. The adult stem cells discovered so far mainly include hematopoietic stem cells, bone marrow mesenchymal stem cells, neural stem cells, liver stem cells, muscle satellite cells, skin epidermal stem cells, intestinal epithelial stem cells, retinal stem cells, pancreatic stem cells, or the like. The induced pluripotent stem cells are pluripotent stem cells obtained by reprogramming terminally differentiated somatic cells by introducing specific transcription factors.

The extracellular vesicles are important vehicles for protein, mRNA, miRNA, and lipid transport to complete the intercellular communication pathway, and are divided into three groups, i.e., exosomes, microvesicles, and apoptotic bodies, according to their size and formation. Among them, exosomes are packaging vesicles with a diameter of approximately 30 to 150 nm, which contain specific proteins, lipids, cytokines, or RNAs.

In this embodiment, the stem cell exosomes are embryonic stem cell exosomes. In other embodiments, the stem cell exosomes are not limited to embryonic stem cell exosomes, but may also be other types of stem cell exosomes, for example, mesenchymal stem cell exosomes.

In an embodiment, the stem cell exosomes are human stem cell exosomes or murine stem cell exosomes. In other embodiments, the stem cell exosomes can also be derived from other animals, such as dogs, cats, etc.

This study confirmed through mouse experiments that the stem cell exosomes can significantly improve the abnormal urination caused by urethral obstruction, can significantly delay the abnormal bladder morphology caused by urethral obstruction, and can be used to treat overactive bladder caused by urethral stricture.

An embodiment of the present disclosure also provides a medicament for treating overactive bladder, the medicament for treating overactive bladder comprising an active ingredient and a pharmaceutically acceptable auxiliary.

The active ingredient refers to any ingredient that provides pharmacological activity or other direct effects in the diagnosis, cure, alleviation, treatment, or prevention of diseases, or affects the structure or any function of human and other animal. Specifically, in this embodiment, the active ingredient includes the stem cell exosomes. In an embodiment, the active ingredient is the stem cell exosomes. In an optional specific example, the active ingredient is at least one of embryonic stem cell exosomes and mesenchymal stem cell exosomes.

In some other embodiments, the stem cell exosomes are not limited to the embryonic stem cell exosomes and the mesenchymal stem cell exosomes as described above, and may also be exosomes secreted by other types of stem cells. It is understandable that, in some embodiments, the active ingredient is not limited to the stem cell exosomes, but may include other ingredients effective in treating overactive bladder. That is, in some other embodiments, the active ingredient of the medicament as described above is a combination of the stem cell exosomes and other active ingredients

The pharmaceutically acceptable auxiliary refers to pharmaceutically acceptable auxiliary materials or carriers that are compatible with other ingredients of the medicament preparation and are suitable for use in contact with the tissues or organs of a subject (for example, humans or animals). The pharmaceutically acceptable auxiliary has no or little toxicity, irritation, allergic reaction, immunogenicity, or other complications during use.

Specifically, the medicament for treating overactive bladder as described above can be oral preparations such as tablets, disintegrating tablets, capsules, granules, pills, oral liquids, or the like, and can also be injection preparations such as injection solutions, freeze-dried powder injections, large infusions, liposome injections, microsphere injections. The medicament can be ordinary preparations, or new or special preparations such as sustained-release preparations, controlled-release preparations, directional preparations, targeted preparations, and nano-preparations.

In an embodiment, the medicament for treating overactive bladder is tablets. In order to prepare the medicament for treating overactive bladder into tablets, various excipients known in the art can be used as the pharmaceutically acceptable auxiliary. Specifically, the medicament for treating overactive bladder is tablets, and the pharmaceutically acceptable auxiliary is at least one selected from the group consisting of a diluent, a binder, a wetting agent, a disintegrant, and a lubricant.

Further, the diluent is at least one selected from the group consisting of starch, dextrin, sucrose, glucose, lactose, mannitol, sorbitol, xylitol, microcrystalline cellulose, calcium sulfate, calcium hydrogen phosphate, and calcium carbonate. The wetting agent is at least one selected from the group consisting of water, ethanol and isopropanol. The binder is at least one selected from the group consisting of starch slurry, dextrin, syrup, honey, glucose solution, microcrystalline cellulose, acacia mucilage, gelatin mucilage, sodium carboxymethyl cellulose, methyl cellulose, hydroxypropyl methyl cellulose, ethyl cellulose, acrylic resin, carbomer, polyvinylpyrrolidone, and polyethylene glycol. The disintegrant is at least one selected from the group consisting of starch, microcrystalline cellulose, low-substituted hydroxypropyl cellulose, cross-linked polyvinylpyrrolidone, cross-linked sodium carboxymethyl cellulose, sodium carboxymethyl starch, polyoxyethylene, sorbitol, fatty acid ester and sodium dodecyl sulfonate. The lubricant is at least one selected from the group consisting of talc, silicon dioxide, stearate, tartaric acid, liquid paraffin and polyethylene glycol. The tablets can also be further prepared into coated tablets, such as sugar-coated tablets, film-coated tablets, enteric-coated tablets, or double-layer tablets and multi-layer tablets.

In an embodiment, the medicament for treating overactive bladder is capsules, or special capsule preparations such as soft capsules, directional capsules, instant capsules, etc. In order to prepare the medicament for treating overactive bladder as described above into capsules, or special capsule preparations such as soft capsules, directional capsules, instant capsules, etc, the above-mentioned stem cell exosomes can be mixed with diluents, glidants, or the like, and then the mixture obtained after mixing can be directly placed in hard capsules, soft capsules or special capsules. In some embodiments, the above-mentioned stem cell exosomes can also be mixed with at least one of a diluent, a binder, and a disintegrant and prepared into particles, pellets, microspheres, liposomes, or the like, which are then placed in hard capsules, soft capsules or special capsules.

In an embodiment, the medicament for treating overactive bladder is injections. In order to prepare the medicament for treating overactive bladder into injections, water, ethanol, isopropanol, propylene glycol, polyethylene glycol or their mixtures can be used as a solvent, and an appropriate amount of the pharmaceutically acceptable auxiliary commonly used in the art can be added and mixed for use.

Specifically, the medicament for treating overactive bladder as described above is injections, and the pharmaceutically acceptable auxiliary is at least one selected from the group consisting of a solubilizer, a pH adjusting agent, and an osmotic pressure regulator. The solubilizer is at least one selected from the group consisting of ethanol, isopropanol, propylene glycol, polyethylene glycol, poloxamer, lecithin, and hydroxypropyl-β-cyclodextrin. The pH adjusting agent is at least one selected from the group consisting of citrate, phosphate, carbonate, acetate, hydrochloric acid, and hydroxide. The osmotic pressure regulator is is at least one selected from the group consisting of sodium chloride, mannitol, glucose, phosphate, citrate, and acetate. As for the preparation of freeze-dried powder injection, mannitol, glucose, and the like can also be added as a proppant. In addition, additives such as coloring agents, preservatives, fragrances, flavoring agents, or the like, if necessary, can also be added to the medicament preparations.

The above-mentioned medicament for treating overactive bladder includes stem cell exosomes. It has been confirmed that the medicament has a good effect on the treatment of overactive bladder syndrome.

### Examples

A detailed description will be given below in conjunction with specific examples. The following examples do not include other components except unavoidable impurities unless specifically stated. The agents and instruments used in the examples are all common in the art unless specifically stated. In the examples, the experimental methods that are not specified with specific conditions are implemented in accordance with conventional conditions, such as the conditions described in the literature, books, or the approach recommended by the manufacturer.

### Example 1

### Preparation and identification of mouse embryonic stem cell exosomes

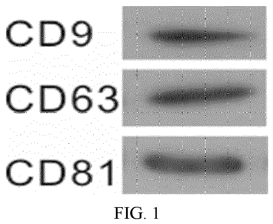

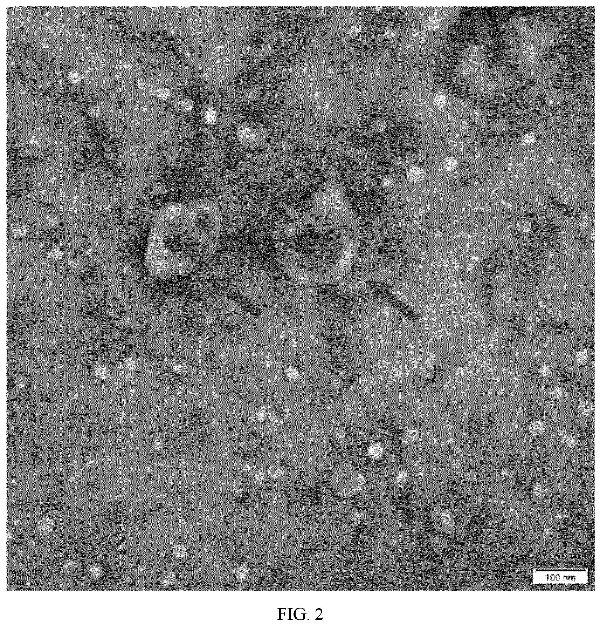

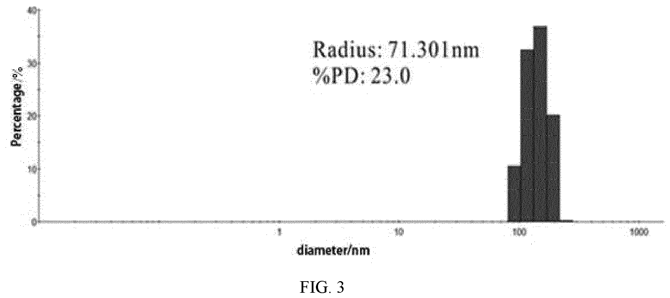

(1) incubation of mouse embryonic stem cells: 20% mitomycin C-treated fibroblasts was incubated in a gelatin-coated dish for 2 hours. After removing the medium and washing the cells with PBS three times, the dish with a feeding layer were obtained. Mouse embryonic stem cells (Jackson Laboratory) were inoculated into the dish with the feeding layer and cultured in H-DMEM medium (2mM L-glutamine, 0.1mM MEM NEAA, 0.1mM β-mercaptoethanol, 0.1mM sodium pyruvate, 1000U/ml LIF and 50 µg/ml penicillin/streptomycin) containing 15% fetal bovine serum.
(2) preparation of mouse embryonic stem cell exosomes: the H-DMEM medium containing 15% fetal bovine serum was centrifuged at 1×10⁵ g for 12 hours at 4°C, and the pellet was removed for use (for purpose of removing exosomes contained in the H-DMEM medium containing 15% fetal bovine serum). After the mouse embryonic stem cells from step (1) were washed with PBS three times, the centrifuged H-DMEM medium containing 15% fetal bovine serum was added to the cells, which was then incubated in an incubator (37°C, 5% CO₂) for 24 hours. Then, the culture medium containing mouse embryonic stem cells was transferred into a centrifuge tube, and centrifuged at 2000 g for 20 minutes at 4°C to pellet the mouse embryonic stem cells. After centrifugation, the supernatant was pipetted into another centrifuge tube, and centrifuged at 1×10⁴ g for 30 minutes at 4°C. The obtained supernatant was pipetted into a high-speed centrifuge tube and centrifuged at 1×10⁵ g at 4°C for 70 minutes. After the supernatant was completely discarded, the resultant pellet was resuspended in 1mL PBS and the resuspended solution was centrifuged at 1×10⁵ g at 4°C for 70 minutes. The supernatant was discarded, the resultant pellet was resuspended in 500 µL PBS, and the resuspended solution was filtered with a 0.22 µm filter to obtain mouse embryonic stem cell exosomes, which were stored at -80°C.
(3) identification of exosomes: western blotting for the identification of CD9, CD81, and CD63, electron microscopy and particle size analysis were used. The results are shown in FIG. 1 to FIG. 3. FIG. 1 is an image showing identification in the western blot, FIG. 2 is an electron micrograph, where the arrows indicate the exosomes; Fig. 3 is a diagram of the particle size distribution, showing that the exosomes has an average particle size of 71.301 nm.

### Example 2

Verification of the effect of mouse embryonic stem cell exosomes on overactive bladder
(1) A mouse model of partial bladder outlet obstruction (PBOO) that has undergone urethral constriction surgery was established to simulate OAB. Specifically, 12-week-old C57BL female mice were randomly divided into a sham operation group and an operation group (that is, a mouse model group of partial bladder outlet obstruction), with 20 mice each in the sham operation group and the operation group. Operation for the operation group includes: the mice were anesthetized with 200 µL of 5% chloral hydrate, and then restrained in the supine position. After the abdominal skin of the mice was depilated, the surrounding area of the urethral orifice was disinfected with 75% ethanol. Then a 26-gauge intravenous indwelling needle was inserted into the bladder through the urethra, the upper skin of the vaginal orifice was cut open, and the proximal urethra was separated. A 7-gauge non-absorbable suture was used to ligate a portion of the urethra near the neck of the bladder, such that the outer tube of the intravenous indwelling needle is tight enough and the probe can be easily pulled out. The intravenous indwelling probe and the outer tube were pulled out successively, and then the incision was closed and the skin was sutured. In the sham operation group, the above-mentioned operation except the urethra ligation were performed in the same site, that is, the urethra was not ligated in the sham operation group.
(2) drug treatment: the mice were injected into the tail vein on days 7 and 10 from the operation and continued feeding. Specifically, ten mice in the operation group were randomly selected, with each of the selected mice injected the mouse embryonic stem cell exosomes prepared in example 1 at a dosage of 0.75 mg/Kg mouse body weight, and named an operation + exosome group. The rest of the mice in the operation group and the mice in the sham operation group were injected with PBS at 0.75 mg/Kg mouse body weight.
(3) Five weeks after the operation, urine stain test, urodynamic test, and bladder weighing were performed on the sham operation group, the operation group, and the operation + exosome group. Details are as follows.

### A. Urine stain test

The following operations were carried out on each mouse in each group: the bedding was removed from the bottom of the mouse cage. After drying up the mouse cage, a 12cm×25cm filter paper was put into the cage and stuck to the bottom of the cage. After adapting the mice for two hours in the cage and removing the filter paper, a new identical filter paper was laid for recording. During this period, the mice were free to access to food but not water. Three hours later, the filter paper was taken out and photographed using the gel imaging system. The photographing parameters are as follows: UV reflected light, aperture: 85, focal length: 50, focus: 100, gain: 35.03dB, contrast: 0, gamma: 1.1, exposure time: 1.1s.

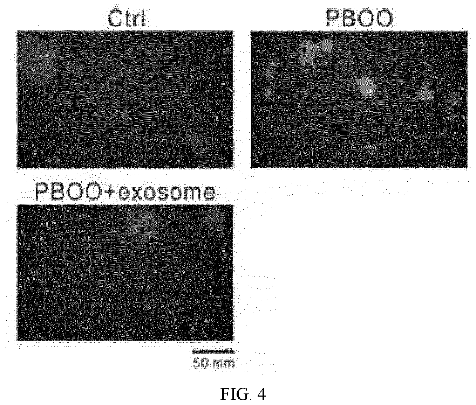

The urine stain images of one mouse in the sham operation group, of one mouse in the operation group, and of one mouse in the operation + exosome group are shown in FIG. 4. In FIG. 4, "Ctrl" represents the sham operation group, "PBOO" represents the operation group, and "PBOO + exosome" represents the operation + exosome group.

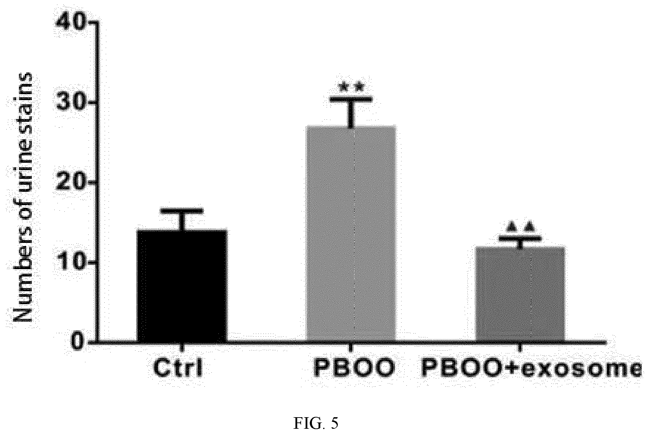

According to statistical analysis, the numbers of urine stains of the mice in the sham operation group, the operation group, and the operation + exosome group were 13.78 ± 2.671 (N=9), 26.75 ± 3.668 (N=8), and 11.70 ± 1.300 (N=10), respectively. The histograms of the numbers of urine stains of the mice in the operation group and the operation + exosome group are shown in FIG. 2. In FIG. 5, "Ctrl" represents the sham operation group, "PBOO" represents the operation group, and "PBOO + exosome" represents the operation + exosome group.

From FIGs. 4 and 5 and statistical data, it can be seen that the number of urine stains of mice in the operation group is much greater than that in the sham operation group and the operation + exosome group, with extremely significant difference, and there is a small and insignificant difference between the numbers of urine stains of mice in the operation + exosome group and the sham operation group, indicating that mouse embryonic stem cell exosomes have therapeutic effects on the mouse model of partial bladder outlet obstruction.

### B. Urodynamic test:

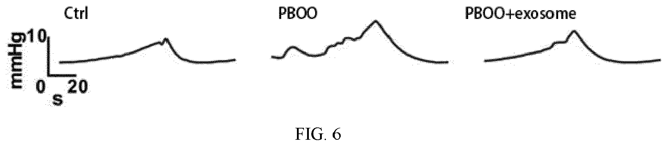

The following operations were carried out on the mice in each group:
1) bladder fistulization and intubation:
   A PE10 tube (with an inner diameter of 0.28 mm and an outer diameter of 0.61 mm) was cut into 35 cm in length, with the end heated to form a cuff-like bulge, which is convenient for fixing in the bladder after fistulization. The tube was sterilized with 75% ethanol and rinsed with normal saline before implantation. The surgical instruments were autoclaved before use. The mice were anesthetized with chloral hydrate, and restrained in the supine position. After the abdominal skin of the mice was depilated and disinfected with 75% ethanol, the abdominal skin was opened. The bladder was fistulized at the opposite tip of the bladder neck with an 18 G needle. Then 3 stitches of purse-string suture were carried out with a 7-0 non-absorbable suture along the outer membrane tightly around the fistula. It should be noted that the smooth muscle layer and the intimal layer cannot be penetrated to prevent bladder leakage. The PE10 tube was implanted through the fistula such that the cuff-like bulge would be inside the bladder. The purse-string suture was tightened and the catheter was gently pulled outward to check the fixation. While 150µL of normal saline was slowly injected into the bladder along the PE10 tube, whether there is any leakage should be carefully checked. After confirming that there is no leakage, the abdominal muscles were sutured with a 3-0 suture, and the PE10 tube was fixed in the upper part to prevent sliding. After placing the mouse in the prone position, the scapula area was depilated, and made a small incision. The hollow gavage needle passed through the skin of the scapula to the abdomen. The PE10 tube was guided to the scapula area through a gavage needle and fixed with a 3-0 suture. The excess catheter was wound into a circle of about 2 cm and fixed with 3-0 sutures (which is to prevent the animal from biting the PE10 tube during the test). Then the mice were placed on the heating plate (about 30 degrees) until they were awake.
2) urodynamic test:
   An urodynamic test was carried out on the mice restored for 3 days after the bladder fistulization and intubation: the mice were placed in the metabolic cage, allowed free activity, and adapted for half an hour before the test began. The PE10 tube in the back of the mouse was connected to a T-branch pipe, which is further connected to a urodynamic instrument (ANDROMEDA) and a micro-injection pump. The bladder perfusion rate was 1.2 mL/h (normal saline), and the urinary dynamics was detected in the awake state. Urodynamic test results were recorded using ANDROMEDA-AUDACT software and analyzed using Excel. The results were expressed as x±s, and differences between the groups were analyzed by t-test and variance. The results are as follows:
   The bladder pressure curves for the sham operation group, the operation group, and the operation + exosome group are shown in FIG. 6.

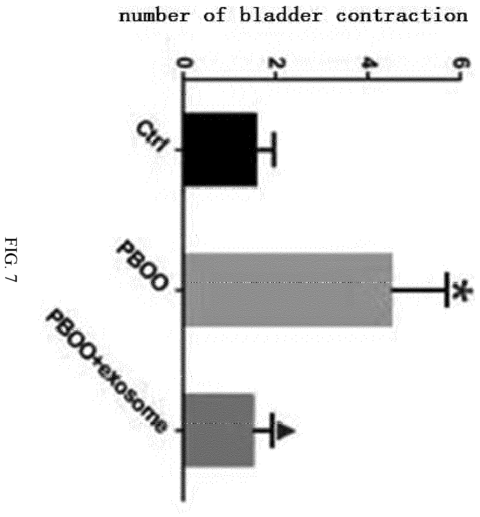

The number of bladder contractions within 30 minutes was 1.556 ± 0.4120 (N=9) for mice in the sham operation group, 4.500 ± 1.210 (N=8) for mice in the operation group and 1.500±0.4282 (N=10) for mice in the operation + exosome group. The histogram of the numbers of bladder contractions within 30 minutes for mice in the sham operation group, the operation group, and the operation + exosome group is shown in FIG. 7. It should be noted that in FIGs. 6 and 7, "Ctrl" represents the sham operation group, "PBOO" represents the operation group, and "PBOO + exosome" represents the operation + exosome group.

From FIGs. 6 and 7 and statistical data, it can be seen that the numbers of the bladder contraction for the mice in the operation + exosome group and the sham operation group have a small difference, and are both much smaller than and significantly different from the number of the bladder contractions for the mice in the operation group, indicating that mouse embryonic stem cell exosomes can reduce the numbers of bladder contractions in the mouse model of partial bladder outlet obstruction.

### C. Bladder status:

After the urodynamic test, the bladders of the mice in each group before and after urination were photographed and weighed, and then the mice were sacrificed. The bladders were collected and were wet-weighed, and the wet bladder/body weight ratio was calculated for mice in each group. The results are as follows:
The photos of the bladder of the mice in the sham operation group, the operation group, and the operation + exosome group before urination are shown in FIG. 6. According to statistical analysis, the wet bladder/body weight ratio (%) was 0.1523 ± 0.007889 for the mice in the sham operation group, 0.3272 ± 0.03525 (N=8) for the mice in the operation group, and 0.1831 ± 0.01153 (N=10) for the mice in the operation + exosome group, respectively. The histogram of the wet bladder/body weight ratios in the sham operation group, the operation group, and the operation + exosome group is shown in FIG.11.

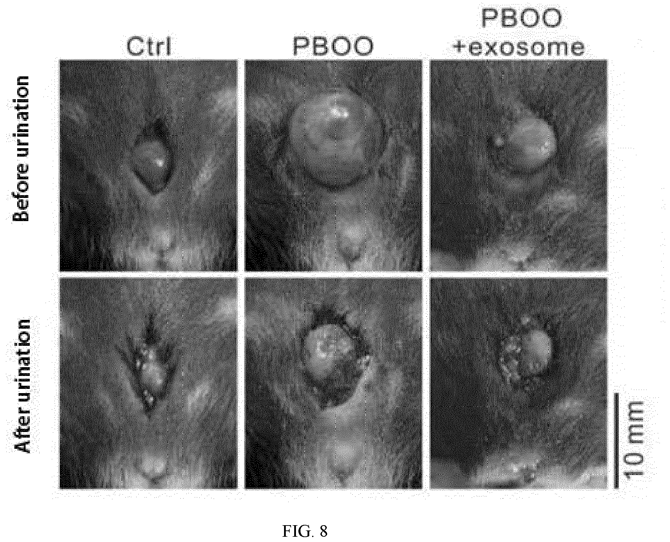

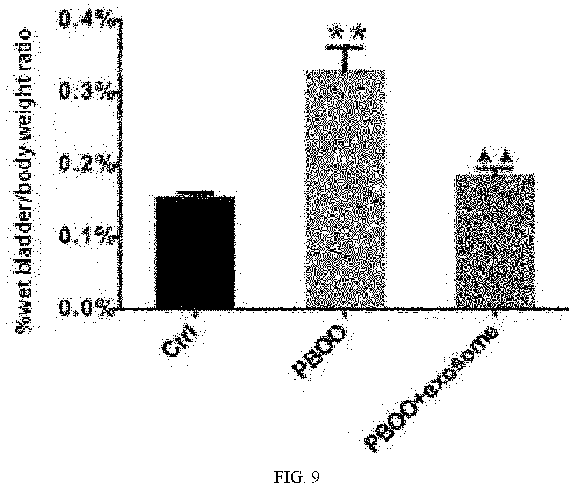

From FIGs. 8 and 9 and statistical data, the bladder exposure of the mice in the operation + exosome group was similar to that of the mice in the sham operation group before and after urination, and both were much smaller than the bladder exposure of the mice in the operation group. The wet bladder/body weight ratio of the mice in the operation + exosome group was similar to the wet bladder/body weight ratio of the mice in the sham operation group, and both were much smaller than the wet bladder/weight ratio of the mice in the operation group. The wet bladder/weight ratio of the mice in the operation group was significantly greater than that in the operation + exosome group and the sham operation group, indicating that the mouse embryonic stem cell exosomes can reduce the wet bladder/weight ratio in the mouse model of partial bladder outlet obstruction.

In summary, the mouse embryonic stem cell exosomes can significantly improve the abnormal urination caused by urethral obstruction, can significantly delay the abnormal bladder morphology caused by urethral obstruction, and can be used to treat overactive bladder caused by urethral stricture.

The various technical features of the above-mentioned embodiments can be combined arbitrarily. To make the description concise, all possible combinations of the various technical features in the above-mentioned embodiments are not described. However, the combinations of these technical features should be regarded as the scope of this specification as long as there is no contradiction in these combinations.

The above-mentioned embodiments only express several embodiments of the present disclosure with more specific and detailed descriptions, but should not be understood as limiting the scope of the disclosure. It should be noted that for those of ordinary skill in the art, several modifications and improvements can be made without departing from the concept of the present disclosure and these all fall within the protection scope of the present disclosure. Therefore, the protection scope of the present disclosure should be subject to the appended claims.

## Claims

1. Stem cell exosomes for use in treatment of overactive bladder.

2. The stem cell exosomes for use according to claim 1, wherein the stem cell exosomes are at least one selected from embryonic stem cell exosomes, mesenchymal stem cell exosomes, and induced pluripotent stem cell exosomes.

3. A medicament for treating overactive bladder, comprising an active ingredient and a pharmaceutically acceptable auxiliary, the active ingredients comprising stem cell exosomes.

4. The medicament according to claim 3, wherein the stem cell exosomes are at least one selected from embryonic stem cell exosomes, mesenchymal stem cell exosomes, and induced pluripotent stem cell exosomes.

5. The medicament according to claim 3, wherein the stem cell exosomes are humanized stem cell exosomes or murine stem cell exosomes.

6. The medicament according to any one of claims 3 to 5, wherein the medicament is in a dosage form of tablets, capsules, granules, pills, injections, or sustained-release preparations.

7. The medicament according to claim 6, wherein the medicament is tablets, and the pharmaceutically acceptable auxiliary is at least one selected from the group consisting of a diluent, a binder, a wetting agent, a disintegrant, and a lubricant.

8. The medicament according to claim 7, wherein the diluent is at least one selected from the group consisting of starch, dextrin, sucrose, glucose, lactose, mannitol, sorbitol, xylitol, microcrystalline cellulose, calcium sulfate, calcium hydrogen phosphate, and calcium carbonate;
and/or, the binder is at least one selected from the group consisting of starch slurry, dextrin, syrup, honey, glucose solution, microcrystalline cellulose, acacia mucilage, gelatin mucilage, sodium carboxymethyl cellulose, methyl cellulose, hydroxypropyl methyl cellulose, ethyl cellulose, acrylic resin, carbomer, polyvinylpyrrolidone, and polyethylene glycol;
and/or, the disintegrant is at least one selected from the group consisting of starch, microcrystalline cellulose, low-substituted hydroxypropyl cellulose, cross-linked polyvinylpyrrolidone, cross-linked sodium carboxymethyl cellulose, sodium carboxymethyl starch, polyoxyethylene, sorbitol, fatty acid ester, and sodium dodecyl sulfonate;
and/or, the lubricant is at least one selected from the group consisting of talc, silicon dioxide, stearate, tartaric acid, liquid paraffin, and polyethylene glycol;
and/or, the wetting agent is at least one selected from the group consisting of water, ethanol, and isopropanol.

9. The medicament according to claim 6, wherein the medicament is injections, and the pharmaceutically acceptable auxiliary is at least one selected from the group consisting of a solubilizer, a pH adjusting agent, and an osmotic pressure regulator.

10. The medicament according to claim 9, wherein the solubilizer is at least one selected from the group consisting of ethanol, isopropanol, propylene glycol, polyethylene glycol, poloxamer, lecithin, and hydroxypropyl-β-cyclodextrin;
and/or, the pH adjusting agent is at least one selected from the group consisting of citrate, phosphate, carbonate, acetate, hydrochloric acid, and hydroxide;
and/or, the osmotic pressure regulator is at least one selected from the group consisting of sodium chloride, mannitol, glucose, phosphate, citrate, and acetate.
